# EUROPEAN PATENT APPLICATION

(11) **EP 2 762 111 A1**
(43) Date of publication of application: **06.08.2014**
(21) Application number: 12837098.8
(22) Date of filing: 17.01.2012
(51) Int. Cl.: A61F 2/82

(54) **INTERVENTIONAL MEDICAL DEVICE AND MANUFACTURING METHOD THEREOF**

(30) Priority: 29.09.2011 CN 201110295357
(71) Applicant: Microport Medical (Shanghai) Co., Ltd., 201203 Shanghai (CN)
(72) Inventor: CAI, Xu, Shanghai 201203 (CN); ZHANG, Dadong, Shanghai 201203 (CN); HU, Yan, Shanghai 201203 (CN); HUANG, Peng, Shanghai 201203 (CN); LI, Junfei, Shanghai 201203 (CN); YUE, Chengyun, Shanghai 201203 (CN); TANG, Zhirong, Shanghai 201203 (CN); LUO, Qiyi, Shanghai 201203 (CN)
(74) Representative: Modiano, Micaela Nadia
(86) International application number: PCT/CN2012/070455
(87) International publication number: WO 2013/044605

(57) **Abstract**

An interventional medical device and manufacturing method thereof, the interventional medical device comprising a stent body (1); the stent body (1) is provided with a drug releasing structure on the surface, the drug in the drug releasing structure being a drug for inhibiting adventitial fibroblast proliferation. When the interventional medical device is implanted into a human body, the drug for inhibiting the adventitial fibroblast proliferation can be slowly released into vessel wall cells in contact with the stent body (1), thus inhibiting the proliferation of the adventitial fibroblasts, promoting vascular compensatory expansion, and reducing the incidence rate of in-stent restenosis.

## Description

### FIELD OF THE INVENTION

The subject application relates to the technical field of medical devices, in particular, to an interventional medical device containing drugs and manufacturing method thereof.

### BACKGROUND ARTS

In recent years, a drug coating was coated onto the stent implanted in the body so as to avoid the the incidence of in-stent restenosis after interventional treatment. Drugs mostly carried by currently used drug-eluting stents are drugs for inhibiting intimal hyperplasia or tunica media hyperplasia, including rapamycin, paclitaxel and derivatives thereof, etc. When the stent carrying the above-mentioned drug is implanted into a human body, the stent will continuously release drugs for inhibiting intimal hyperplasia or tunica media hyperplasia to the vessel wall to reduce the incidence rate of in-stent restenosis.

Studies have shown that vascular restenosis formation is not only related to intimal hyperplasia or tunica media hyperplasia after vascular injury but also to vascular remodelling. Vascular remodeling is the main factor that casues in-stent restenosis, accounting for 70% possible causes of restenosis, while intimal hyperplasia or tunica media hyperplasia accounts for only 30% possible causes of restenosis.

Therefore, the current drug-eluting stents for inhibiting intimal hyperplasia or tunica media hyperplasia can not reduce the incidence of in-stent restenosis to the greatest extent. In addition, inhibition of intimal hyperplasia or tunica media hyperplasia may delay vascular endothelialisation, and the problem that blood vessels can not be completely endothelialized may cause late thrombosis.

### SUMMARY OF THE INVENTION

In view thereof, the examples of the present application provide an interventional medical device and manufacturing method thereof. The interventional medical device promotes vascular compensatory expansion by inhibiting the proliferation of adventitial fibroblasts, so as to reduce the incidence rate of in-stent restenosis.

In order to achieve the above objects, the examples of the present application provide the following technical solutions:

An interventional medical device comprising a stent body with a drug releasing structure on its surface, wherein the drug in the drug releasing structure is a drug for inhibiting adventitial fibroblast proliferation.

Preferably, the drug releasing structure is a dense mixed layer formed by a polymer and the drug inhibiting adventitial fibroblast proliferation.

Preferably, the polymer includes polylactic acid, polyethylene glycol, styrene-butene copolymer, polycaprolactone, poly(butyl methacrylate), poly (ethyl methacrylate), polyvinyl ethyl acetate, polyurethane, polyvinyl pyrrolidone, polyphosphorylcholine, silk protein, gelatin, chitin and/or hyaluronic acid.

Preferably, the drug releasing structure is a microporous structure prepared on the surface of the stent body or a microporous coating structure formed on the surface of the stent body, and the drug is loaded in the microporous structure or microporous coating structure.

Preferably, the drug for inhibiting adventitial fibroblast proliferation includes at least one drug selected from group consisting of tanshinone, asiaticoside, madecassoside, ligustrazine, dracorhodin, Rosuvastatin, and angiotensin.

Preferably, the stent body comprises coronary artery stent, intracranial vascular stent, peripheral vascular stent, intraoperative stent, heart valve stent, biliary tract stent, esophageal stent, intestinal tract stent, pancreatic duct stent, urethral stent or tracheal stent.

A method for preparing an interventional medical device, comprising:

Preparing microporous structures on the surface of a stent body;

Formulating a solution containing a drug for inhibiting adventitial fibroblast proliferation;

Loading the drug within the formulated solution into the microporous structure;

Drying the stent body to obtain the interventional medical device.

Preferably, preparing microporous structures on the surface of a stent body comprises forming micropores on the surface of the stent body by anodic oxidation, micro-arc oxidation and/or chemical corrosion.

Preferably, preparing microporous structures on the surface of a stent body comprises preparing a coating having micropores on the surface of the stent body.

Preferably, loading the drug within the formulated solution into the microporous structure comprises loading the drug within the solution into the micropores or coatings having micropores by ultrasonic spraying, air spraying and/or dipping.

A method for preparing an interventional medical device, comprising:

Formulating a mixed solution of a drug inhibiting adventitial fibroblast proliferation and a polymer;

Coating the surface of the stent body with the mixed solution;

Drying the stent body to obtain the interventional medical device.

Preferably, the coating step comprises ultrasonic spraying, air spraying and/or dipping.

It can be seen from the above technical solutions that, when the interventional medical device is used, the drug carried thereon for inhibiting adventitial fibroblast proliferation can be slowly released into vessel wall cells in contact with the stent body after it is implanted into a human body, thus inhibiting the proliferation of the adventitial fibroblasts, functioning in vascular remodeling by blocking fibroblast proliferation, promoting compensatory expansion of the damaged blood vessel, thereby reducing the incidence rate of in-stent restenosis.

In addition, compared to the current drug-eluting stents using rapamycin, paclitaxel and derivatives thereof, the interventional medical device provided by the examples of the present application not only has low inhibition rate on endothelial cells, but also promotes endothelial cell growth and accelerates the process of endothelialization.

### ILLUSTRATION

In order to more clearly illustrate the technical solutions of the examples of the present application or the prior art, the accompanying drawings which are required to be used in the description of the examples or the prior art will be briefly introduced below. It is apparent that the accompanying drawings in the following description are merely some examples described in the present application. For those of ordinary skill in the art, it is also possible to derive other drawings according to these drawings without creative efforts.
Figure 1 is a structural schematic diagram of a specific embodiment of the interventional medical device provided by the present application;
Figure 2 is a statistical chart about an inhibition rate of asiaticoside, paclitaxel and rapamycin on human umbilical vein endothelial cells provided by the present application;
Figure 3 is a structural schematic diagram of another specific embodiment of the interventional medical device provided by the present application;
Figure 4 is structural schematic diagram of another specific embodiment of the interventional medical device provided by the present application;
Figure 5 is a technological process of the method for preparing the interventional medical device provided by the present application;
Figure 6 is another technological process of the method for preparing the interventional medical device provided by the present application;
Figure 7 is another technological process of the method for preparing the interventional medical device provided by the present application.

### DETAILED DESCRIPTION OF THE INVENTION

In order to make those skilled in the art better understand technical solutions of the present application, the technical solutions of the examples of the present application will be clearly and fully described below by making reference to the accompanying drawings of the examples of the present application. Obviously, the described examples are merely a part of the examples of the present application, but not all examples. Based on the examples of the present application, all other examples obtained by those of ordinary skill in the art without creative efforts should fall within the protection scope of the present application.

The examples of the present application provide an interventional medical device comprising a stent body with a drug releasing structure on its surface, and the drug in the drug releasing structure is a drug for inhibiting adventitial fibroblast proliferation.

An example:

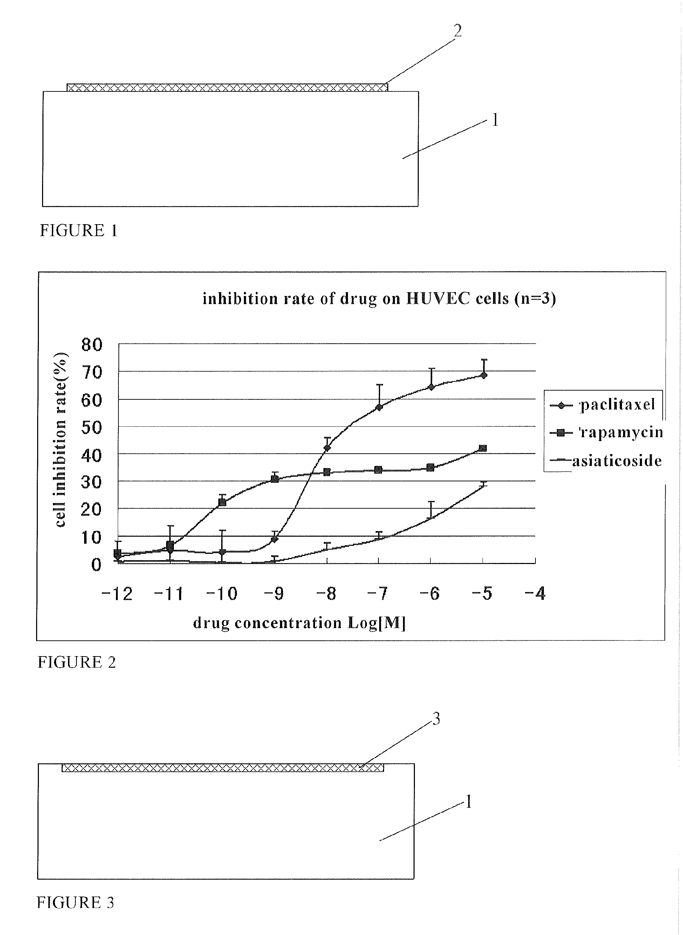

Figure 1 is a structural schematic diagram of a specific embodiment of the interventional medical device provided by the present application.

As shown in Figure 1, 1 indicates a stent body and 2 indicates a drug releasing coating. Drug releasing coating 2 is coated on the outer surface of stent body 1, wherein:
Stent body 1 can be a coronary artery stent, intracranial vascular stent, peripheral vascular stent, intraoperative stent, heart valve stent, biliary tract stent, esophageal stent, intestinal tract stent, pancreatic duct stent, urethral stent or tracheal stent. Further, the material of stent body 1 can be a material with good biocompatibility and mechanical characteristics, such as stainless steel, cobalt-based alloy, nickel-based alloy, titanium alloy, degradable magnesium alloy or a polymer, etc.

Drug releasing coating 2 is a dense mixed layer formed by a polymer and a drug inhibiting adventitial fibroblast proliferation. That is, drug releasing coating 2 is used as a carrier to allow the surface of stent body 1 to carry drugs for inhibiting adventitial fibroblast proliferation. Drug for inhibiting adventitial fibroblast proliferation includes at least one drug selected from the group consisting of tanshinone, asiaticoside, madecassoside, ligustrazine, dracorhodin, Rosuvastatin, and angiotensin. In the example of the present application, asiaticoside is preferred. In addition, the polymer in drug releasing coating 2 can be a polymer having biocompatibility and controlled release properties, for example, polylactic acid, polyethylene glycol, styrene-butene copolymer, polycaprolactone, poly(butyl methacrylate), poly (ethyl methacrylate), polyvinyl ethyl acetate, polyurethane, polyvinyl pyrrolidone, polyphosphorylcholine, silk protein, gelatin, chitin and/or hyaluronic acid.

Asiaticoside is the total glycosides extracted from Umbelliferae *Centella asiatica.* Asiaticoside can inhibit the pathological role of TGF-beta by increasing expression of Smad7 that inhibits Smad transduction signal, thereby functioning in vascular remodeling by blocking fibroblast proliferation, promoting vascular compensatory expansion, thus reducing the incidence rate of in-stent restenosis.

Furthermore, in *in vitro* cell assays, HUVEC (Human Umbilical Vein Endothelial Cells) were used to test the inhibition rate of asiaticoside, paclitaxel and rapamycin on HUVEC, respectively. Figure 2 shows the statistical chart of inhibition rates for asiaticoside, paclitaxel and rapamycin on HUVEC provided by the present application. It can be seen from Figure 2 that the inhibition rate of asiaticoside on HUVEC was significantly lower than those of paclitaxel and rapamycin, and its concentration was within the range of 10⁻¹²-10⁻⁹ M. Asiaticoside almost had no inhibitory effect on HUVEC.

Meanwhile, studies have found that asiaticoside could also promote endothelial cell growth and accelerate endothelialization process. For detailed, see "Experimental study of the effect of asiaticoside on preventing restenosis after percutaneous coronary intervention (CLC R541.4 Article ID: 1671-8259 (2005) 05-0477-03).

Thus it can be seen that, compared with the current drug-eluting stents using rapamycin, paclitaxel and derivatives thereof, the interventional medical device provided by the examples of the present application not only has low inhibition rate on endothelial cells, but also promotes endothelial cell growth and accelerates the process of endothelialization.

### Another Example:

Figure 3 is a structural schematic diagram of another specific embodiment of the interventional medical device provided by the present application.

As shown in Figure 3, 1 indicates a stent body, and 3 indicates micropores formed on the surface of the stent.

In the example of the present application, the drug releasing structure is micropore 3, which can be obtained by oxidating or eroding the surface of stent body 1. Micropore 3 can be loaded with drugs for inhibiting adventitial fibroblast proliferation, thus stent body 1 will carry drugs for inhibiting adventitial fibroblast proliferation on its surface.

### A further Example:

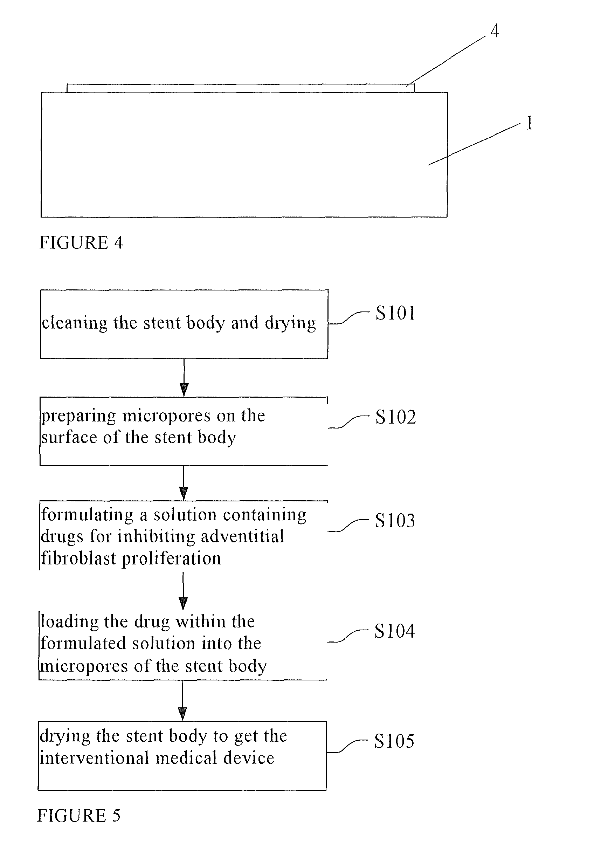

Figure 4 is structural schematic diagram of another specific embodiment of the interventional medical device provided by the present application.

In the interventional medical device shown in Figure 3, micropore 3 is obtained by directly oxidating or eroding the surface of stent body 1. However, in the example of the present application, a layer of microporous coating can be prepared on the surface of stent body 1. As shown in Figure 3, 1 indicates a stent body, and 4 indicates a microporous coating. This eliminates the need for oxidation or corrosion of the surface of stent main body 1, but directly prepares microporous coating 4 on the surface of stent body 1 to obtain micropores loaded with drugs.

### A further Example:

Figure 5 is a technological process of the preparation method of the interventional medical device provided by the present application.

As shown in Figure 5, in the example of the present application, taking metal stent as the example of the stent body, the preparation method of the interventional medical device comprises:

### Step S101: cleaning the stent body and drying.

During the preparation of the interventional medical device, in order to avoid the impact of residual stains from the stent body on the quality of interventional medical device, it is necessary to clean the stent body first.

Step S102: preparing micropores on the surface of the stent body.

Micropores on the surface of the stent body are formed by electrochemical corrosion and/or chemical corrosion, in which electrochemical corrosion includes anodic oxidation, micro-arc oxidation and so on. Micropores can be formed on the surface of the stent body by this step. Figure 2 shows their structural schematic diagram.

Step S103: formulating a solution containing drugs for inhibiting adventitial fibroblast proliferation.

In the example of the present application, the drug for inhibiting adventitial fibroblast proliferation is preferably asiaticoside. When formulating, 50mg asiaticoside is dissolved in 10 ml ethanol solution and the mixture is mixed thoroughly.

Step S104: loading the drug within the formulated solution into the micropores of the stent body.

The stent body with micropores on its surface obtained in step S102 is immersed into the solution formulated in step S103, so that the drugs within the solution can be loaded into the micropores on the surface of the stent body.

Step S105: Drying the stent body to get the interventional medical device.

### A further Example:

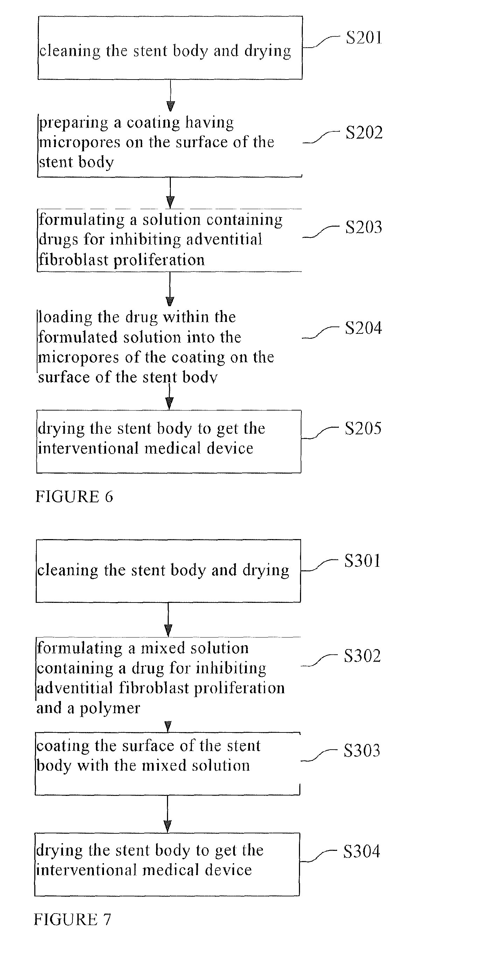

Figure 6 is another technological process of the preparation method of the interventional medical device provided by the present application.

As shown in Figure 6, in the example of the present application, the preparation method of the interventional medical device comprises:

### Step S201: cleaning the stent body and drying.

Step S202: preparing a coating having micropores on the surface of the stent body.

Particular process includes the following steps: the silk protein solution is uniformly coated on the surface of the stent body. Then the stent body is subject to thermal or chemical denaturation, and infiltration by pure water. After that, the stent body is freezed and the termperature is increased to dry the body. A coating with microporous structure is thus formed on the surface of the stent body.

Step S203: formulating a solution containing drugs for inhibiting adventitial fibroblast proliferation.

In the example of the present application, the drug for inhibiting adventitial fibroblast proliferation is preferably asiaticoside. When formulating, 50mg asiaticoside is dissolved in 10 ml ethanol solution and mixed thoroughly.

Step S204: loading the drug within the formulated solution into the micropores of the coating on the surface of the stent body.

The stent body with microporous coating on its surface obtained in step S202 is immersed into the formulated solution, so that the drug within the solution can be loaded into the micropores of the coating on the surface of the stent body.

Step S205: Drying the stent body to get the interventional medical device.

### A further Example:

Figure 7 is another technological process of the preparation method of the interventional medical device provided by the present application.

As shown in Figure 7, in the example of the present application, the preparation method of the interventional medical device comprises:

Step S301: cleaning the stent body and drying.

Step S302: formulating a mixed solution containing a drug for inhibiting adventitial fibroblast proliferation and a polymer.

In the example of the present application, the polymer is polylactic acid and the drug for inhibiting adventitial fibroblast proliferation is preferably asiaticoside. A solution in which polylactic acid and asiaticoside are present in a ratio in the range from 1:1 to 1:4 is formulated. For example, 10mg asiaticoside and 20mg poly(lactic acid) are added to 10ml tetrahydrofuran. After they are sufficiently dissolved, the mixture is mixed uniformly.

Step S303: coating the surface of the stent body with the mixed solution.

In the example of the present application, the mixed solution formulated in step 302 can be coated to the stent body by ultrasonic spraying, air spraying or dipping.

Step S304: Drying the stent body to get the interventional medical device.

The above examples are only preferred embodiments of the present application. With these examples the skilled person can understand or realize the present application. Various modifications to these examples will be apparent to the skilled person in the art, and the generic principles defined herein may be implemented in other examples without departing from the spirit or scope of the present application. Accordingly, the present application will not be limited to these examples described herein, but meet the widest scope consistent with the principles and novel features disclosed herein.

## Claims

1. An interventional medical device comprising a stent body with a drug releasing structure on its surface, wherein the drug in said drug releasing structure is a drug for inhibiting adventitial fibroblast proliferation.

2. The interventional medical device according to claim 1, wherein said drug releasing structure is a dense mixed layer formed by a polymer and the drug for inhibiting adventitial fibroblast proliferation.

3. The interventional medical device according to claim 2, wherein said polymer includes polylactic acid, polyethylene glycol, styrene-butene copolymer, polycaprolactone, poly(butyl methacrylate), poly (ethyl methacrylate), polyvinyl ethyl acetate, polyurethane, polyvinyl pyrrolidone, polyphosphorylcholine, silk protein, gelatin, chitin and/or hyaluronic acid.

4. The interventional medical device according to claim 1, wherein said drug releasing structure is a microporous structure prepared on the surface of said stent body or a microporous coating structure formed on the surface of said stent body, and the drug is loaded in said microporous structure or microporous coating structure.

5. The interventional medical device according to claim 1, wherein said drug for inhibiting adventitial fibroblast proliferation includes at least one drug selected from the group consisting of tanshinone, asiaticoside, madecassoside, ligustrazine, dracorhodin, Rosuvastatin, and angiotensin.

6. The interventional medical device according to claim 1, wherein said stent body is selected from the group consisting of coronary artery stent, intracranial vascular stent, peripheral vascular stent, intraoperative stent, heart valve stent, biliary tract stent, esophageal stent, intestinal tract stent, pancreatic duct stent, urethral stent or tracheal stent.

7. A method for preparing an interventional medical device, wherein said method comprises:
Preparing microporous structures on the surface of a stent body;
Formulating a solution containing a drug which inhibits adventitial fibroblast proliferation;
Loading the drug within the formulated solution into said microporous structures;
Drying said stent body, thereby obtaining the interventional medical device.

8. The method according to claim 7, wherein preparing microporous structures on the surface of the stent body comprises forming micropores on the surface of the stent body by anodic oxidation, micro-arc oxidation and/or chemical corrosion.

9. The method according to claim 7, wherein preparing microporous structures on the surface of the stent body comprises preparing a coating having micropores on the surface of said stent body.

10. The method according to claim 7, wherein, loading the drug within the formulated solution into said microporous structure comprises loading the drug within said solution into said microporous structure by ultrasonic spraying, air spraying and/or dipping.

11. A method for preparing an interventional medical device, wherein said method comprises:
Formulating a mixed solution of a drug inhibiting adventitial fibroblast proliferation and a polymer;
Coating the surface of the stent body with the mixed solution;
Drying the stent body to obtain said interventional medical device.

12. The method according to claim 11, wherein said coating comprises ultrasonic spraying, air spraying and/or dipping.
